(19) Europäisches Patentamt
European Patent Office
Office européen des brevets

(11) Veröffentlichungsnummer: **0 591 809 A2**

(12) # EUROPÄISCHE PATENTANMELDUNG

(21) Anmeldenummer: **93115566.7**

(22) Anmeldetag: **27.09.93**

(51) Int. Cl.[5]: **C07D 519/00**, C07H 21/00, C07D 307/58, C08F 4/04, C08F 4/34, //(C07D519/00, 495:00,495:00)

(30) Priorität: **09.10.92 DE 4234074**
**09.07.93 DE 4322885**

(43) Veröffentlichungstag der Anmeldung:
**13.04.94 Patentblatt 94/15**

(84) Benannte Vertragsstaaten:
**BE CH DE DK ES FR GB IT LI NL SE**

(71) Anmelder: **BAYER AG**

**D-51368 Leverkusen(DE)**

(72) Erfinder: **Heiliger, Ludger, Dr.**
**Carl-Rumpff-Strasse 8**
**D-51373 Leverkusen(DE)**

(54) **Biologisch aktive Initiatoren für die radikalische Polymerisation.**

(57) Es wurden biologisch aktive Initiatoren (Radikalkettenstarter) gefunden, die den allgemeinen Aufbau der Formel (I) aufweisen

$A—L—B[L—A]_y$ (I),

worin

A ein biologisch aktiver Teil,
B ein radikalbildender Teil,
L eine Linkergruppierung und
y die Zahl 0 oder 1, bevorzugt 1, bedeuten.

Als biologisch aktiver Teil A kommt beispielsweise Biotin, Digitoxin, Digoxin, Digitoxigenin, Digoxigenin und Oligonukleotide aus 1 bis 80, vorzugsweise 15 bis 50 und insbesondere 20 bis 35 Nukleotidbausteinen, infrage.

EP 0 591 809 A2

Es wurden biologisch aktive Initiatoren (Radikalkettenstarter) gefunden, die den allgemeinen Aufbau der Formel (I) aufweisen

$A{-}L{-}B[L{-}A]_y$ (I),

worin

- A ein biologisch aktiver Teil,
- B ein radikalbildender Teil,
- L eine Linkergruppierung und
- y die Zahl 0 oder 1, bevorzugt 1, bedeuten.

Als biologisch aktiver Teil A kommt beispielsweise Biotin, Digitoxin, Digoxin, Digitoxigenin, Digoxigenin und Oligonukleotide aus 1 bis 80, vorzugsweise 15 bis 50 und insbesondere 20 bis 35 Nukleotidbausteinen, infrage.

Biotin, Digoxigenin und Oligonukleotid aus 15 bis 50, insbesondere 20 bis 35 Nukleotidbausteinen sind bevorzugt.

Als Linkergruppierung L kommen folgende Gruppierungen infrage:
$-SO_2-$, -COO-, $-SO_2NH-$, -CO-NH-, -NH-CO-O-, NH-CS-O-, -NH-CO-NH-, -NH-CS-NH-, -O-, -NH-, -S-.

Bevorzugte Linkergruppierungen sind -CO-NH-, -NH-CO-NH-, -COO-, -NH-CO-O. -CO-NH- und -NH-CO-NH sind besonders bevorzugt.

Die linkergruppierung L verknüpft den biologisch aktiven Teil und den radikalbildenden Teil B kovalent miteinander.

Falls eine erhöhte Beweglichkeit der Bausteine A und/oder B erwünscht ist, kann durch L auch eine Abstandshalterfunktion ausgeübt werden, wobei L dann aus folgenden Untereinheiten der Formel (IV) bestehen kann:

$L^1 - R - L^1$ (IV),

worin

- $L^1$ die bei L angegebenen Bedeutungen hat und
- R für $C_1$-$C_{20}$-Alkylen, vorzugsweise $C_3$-$C_{15}$-Alkylen, besonders bevorzugt für $C_5$-$C_{10}$-Alkylen, für $C_6$-$C_{10}$-Arylen-$C_2$-$C_{10}$-Alkylen, vorzugsweise für Phenylen- bzw. Naphthylen-$C_2$-$C_8$-Alkylen, für $(CH_2$-$CH_2$-$O)_n$ steht, worin
- n 1 bis 20, vorzugsweise 3 bis 15 und besonders bevorzugt 3 bis 10, bedeutet.

Als radikalbildender Teil B kommen folgende Verbindungen infrage:

1) Azostrukturen der allgemeinen Formel (V)

$R^{11} - N = N - R^{12}$ (V),

worin

$R^{11}$ und $R^{12}$ $C_1$-$C_{20}$-Alkyl, $C_3$-$C_{10}$-Cycloalkyl, $C_7$-$C_{20}$-Aralkyl oder die Gruppe

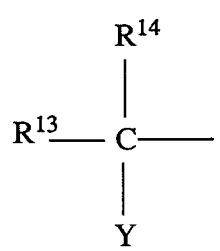

bedeuten, und

Y CN, $N_3$ oder

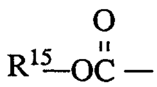 , 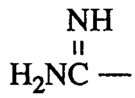 , 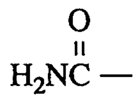 , 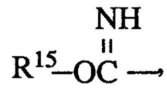

$R^{15}$-O- ,

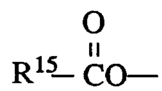 ,

$R^{15}$S- , NCS- , SCN- ,

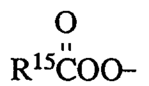 ,

HO- ,

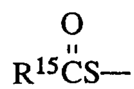 ,

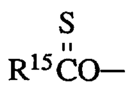 , 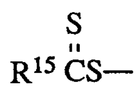 , 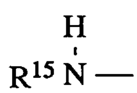 oder 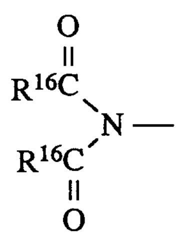

bedeutet,

$R^{13}$, $R^{14}$ und $R^{15}$ unabhängig voneinander $C_1$-$C_{20}$-Alkyl, $C_3$-$C_6$-Cycloalkyl bedeuten oder wenn $R^{13}$ und $R^{14}$ verknüpft sind $C_2$-$C_{30}$-Alkylen bedeuten oder zusätzlich einer der Reste $R^{13}$ oder $R^{14}$, jedoch nicht beide gleichzeitig, Phenyl, Tolyl, Xylyl, Benzyl oder Phenethyl bedeutet,

$R^{16}$ unabhängig $C_1$-$C_6$-Alkyl, $C_3$-$C_6$-Cycloalkyl oder $C_6$-$C_{12}$-Aryl bedeutet;

2) Tetraaryl/alkylethane der allgemeinen Formel (VI)

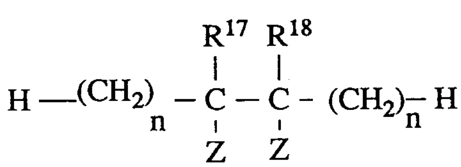 (VI),

in welcher

Z für Hydroxy, $C_1$-$C_6$-Alkyl oder $C_6$-$C_{20}$-Aryl, insbesondere für Hydroxy, Methyl, Ethyl, n- oder iso-Propyl, Phenyl oder Naphthyl steht,

$R^{17}$ und $R^{18}$ unabhängig voneinander für $C_1$-$C_6$-Alkyl oder $C_6$-$C_{20}$-Naphthyl, insbesondere für Methyl, Ethyl, n- oder iso-Propyl, Phenyl oder Naphthyl stehen und

n für die Zahlen 1 bis 6, vorzugsweise 1, 2, 3, 4 oder 5, steht;

3) Dinitrile der Formel (VII)

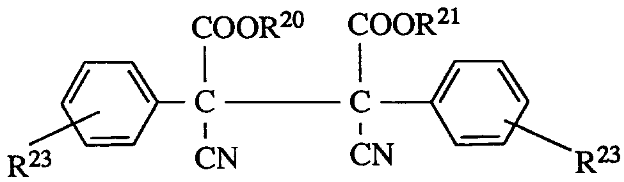 (VII),

in welcher

$R^{20}$, $R^{21}$ und $R^{23}$ unabhängig voneinander für

$$(CH_2)_mH$$

stehen,

worin

m für die Zahlen 1 bis 6, vorzugsweise 1, 2, 3, 4 oder 5 steht;

4) Peroxide der allgemeinen Formel (VIII)

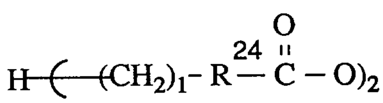 (VIII),

in welcher

l die Zahlen 0 bis 6, vorzugsweise 0, 1, 2, 3 oder 4, insbesondere 0, 1 oder 2, bedeutet und

$R^{24}$ Phenylen, Naphthylen, $C_3$-$C_6$-Alkylen oder $C_3$-$C_6$-Cycloalkylen bedeutet.

Die unter 1) und 4) genannten Strukturen der Bausteine B sind bevorzugt.

Die Verbindungen der Formeln (V) bis (VIII) sind allgemein bekannt (vgl. beispielsweise US-PS 3 956 269, Houben-Weyl, Makromolekulare Stoffe, Teil 1, S.16-19).

Bevorzugte Azostrukturen der Formel (V) sind Verbindungen der Formel (Va)

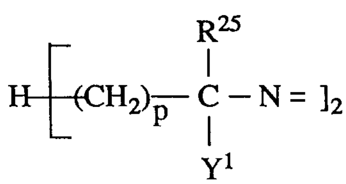 (Va),

in welcher

p die Zahlen 1 bis 20, vorzugsweise 1 bis 15, insbesondere 2 bis 10, bedeutet,

$Y^1$ für CN, $N_3$, $COOR^{26}$ und

$R^{25}$ und $R^{26}$ unabhängig voneinander $C_1$-$C_6$-Alkyl, insbesondere Methyl, Ethyl, n- oder iso-Propyl, oder $C_3$-$C_6$-Cycloalkyl, insbesondere Cyclopropyl, Cyclopentyl oder Cyclohexyl, bedeuten.

Eine besonders bevorzugte Struktur der unter 1) genannten Bausteine B hat die Formel (Vb)

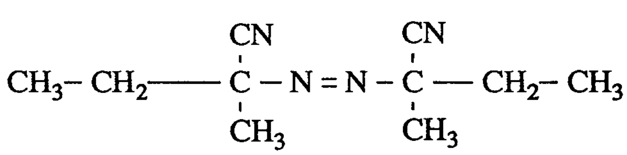 (Vb),

d.h., p bedeutet 2, $R^{25}$ $CH_3$ und $Y^1$ CN.

Eine besonders bevorzugte Struktur der unter 4) genannten Bausteine B entspricht der Formel (VIII) mit 1 = O und $R^{24}$ = Phenylen.

Die Strukturen der Formeln (V) bis (VIII), die den radikalbildenden Teil ausmachen, tragen 1 (y in Formel (I) = 0) oder 2 (y in Formel (I) = 2) reaktionsfähige Gruppen $X^1$ (vgl. Beschreibung der Formel (IX).

In den Azostrukturen der Formel (V) tragen die Reste $R^{11}$ und $R^{12}$ diese Gruppe (n). In den Strukturen der Formeln (Va), (Vb), (VI) und (VIII) sind die endständigen Wasserstoffatome durch diese Gruppe(n) ersetzt. Die Dinitrile der Formel (VII) tragen diese Gruppe(n) symmetrisch um die zentrale Bindung in $R^{20}$, $R^{21}$ und/oder $R^{23}$.

Im einzelnen seien beispielsweise die folgenden biologisch aktiven Initiatoren der Formel (I) aufgezeigt.

Initiator 1:

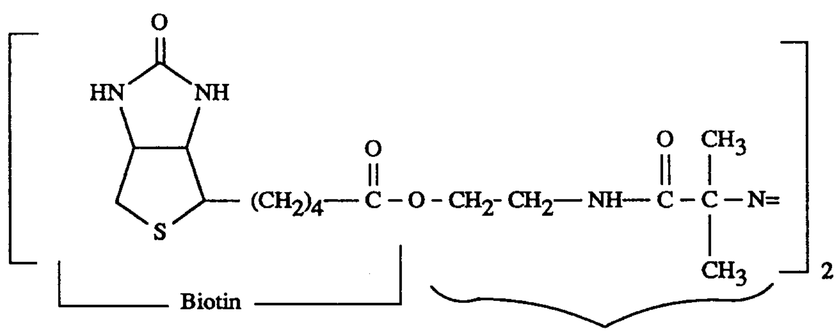

biologisch aktiver Teil A radikalbildender Teil B

Initiator 2:

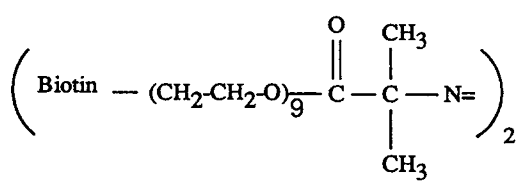

Initiator 3:

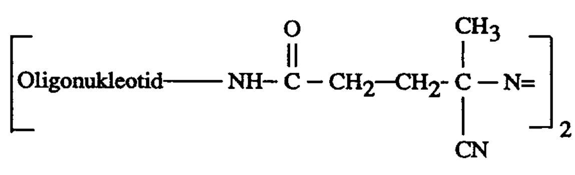

Initiator 4:

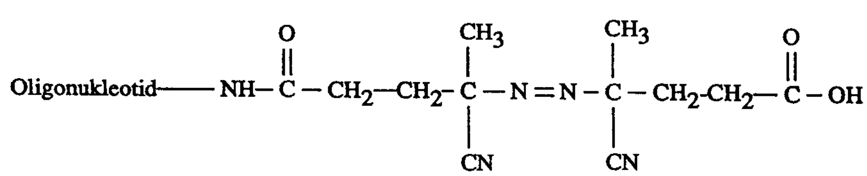

Initiator 5:

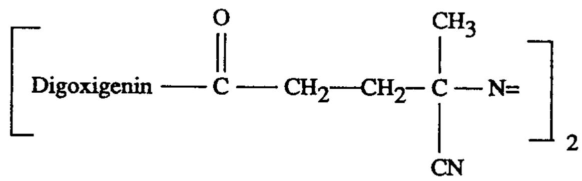

Initiator 6:

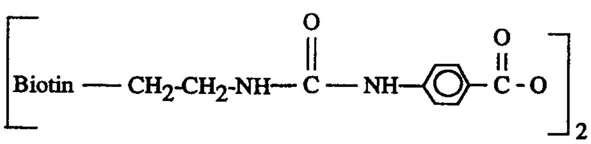

Es wurde weiterhin ein Verfahren zur Herstellung von biologisch aktiven Radikalketteninitiatoren der allgemeinen Formel (I)

$A—L—B[L—A]_y$ (I)

gefunden, dadurch gekennzeichnet, daß man radikalbildende Verbindungen der allgemeinen Formel (IX)

$X^1—B(X^1)_y$ (IX),

wobei

- B wie oben definiert ist und
- $X^1$ NCO, NCS, COCl, COOH, CO-O-N-Hydroxysuccinimid, OH, $NH_2$, SH, Cl, Br oder I sein kann und
- y 0 oder 1, bevorzugt 1 ist,

mit 1 oder 2 Äquivalenten biologisch aktiven Substanzen A in gegenüber den unter $X^1$ genannten Gruppierungen chemisch inerten Lösungsmitteln, wie beispielsweise Chloraliphaten, Ketone, Nitrile, Sulfoxide, Sulfone u.ä., bei Temperaturen zwischen 0 und 40 °C umsetzt.

Bei Verbindungen der allgemeinen Formel (IX),

$X^1—B(X^1)_y$ (IX),

in denen

- $X^1$ COCl oder $CONHSO_2Cl$ bedeutet,

ist dem Reaktionsgemisch zweckmäßigerweise ein Protonenfänger wie beispielsweise Pyridin oder Triethylamin zuzusetzen, wohingegen, wenn X = COOH, die Umsetzung in Gegenwart von Carbodiimiden, beispielsweise Dicyclohexylcarbodiimid, durchgeführt wird.

Als Verbindung der Formel $X^1—B(X^1)_y$ werden in das erfindungsgemäße Verfahren bevorzugt Verbindungen, in denen $X^1$ = NCO und CO-O-N-Hydroxysuccinimid bedeutet, eingesetzt.

Die Reaktion erfolgt bevorzugt zwischen 0 und 30 °C, besonders bevorzugt zwischen 15 und 25 °C und insbesondere bei ca. 20 °C. Als Lösungsmittel werden bevorzugt Methylenchlorid, Aceton oder Acetonitril eingesetzt.

Die Verbindungen der Formel (IX) sind allgemein bekannt oder lassen sich nach allgemein bekannten Verfahren herstellen (vgl. z.B. US-PS 4 155 937).

Die Linkergruppierung L wird gebildet durch die Umsetzung von $X^1$ aus der Formel $X^1—B(X^1)_y$ mit der reaktiven Gruppe im biologisch aktiven Baustein A, beispielsweise der Carboxylgruppe in Biotin oder der Aminogruppe im Oligonukleotid.

Die Isolierung der biologisch aktiven Radikalkettenstarter erfolgt nach an sich bekannten Methoden, z.B. nach dem Abfiltrieren von gegebenenfalls gebildeten (ionischen) Nebenprodukten durch Abdampfen des Lösungsmittels im Hochvakuum, wenn niedrigsiedende Lösungsmittel verwendet werden, oder durch Ausfällung durch Zusatz eines geeigneten Fällungsmittels, wobei das erfindungsgemäße Produkt in der Regel rein anfällt. In den Fällen, in denen die Nebenprodukte nicht flüchtig oder nicht durch Umlösen oder Filtrieren von den erfindungsgemäßen Verbindungen abtrennbar sind, erfolgt die Isolierung der erfindungsgemäßen Verbindungen durch an sich bekannte Methoden der Flüssigchromatographie, beispielsweise Säulenchromatographie oder präparative HPLC (Hochdruckflüssigkeitschromatographie).

Die erfindungsgemäßen Verbindungen sind z.B. geeignet als Initiatoren für radikalische Polymerisationen. Bei Verwendung als Radikalstarter können deren Zerfallsprodukte in die hergestellten Polymere als Endgruppen eingebaut werden. Die somit Endgruppen modifizierten Polymere (Markierungspolymere) lassen sich selektiv z.B. mit Substraten umsetzen, die einer biochemischen Erkennungsreaktion zugänglich sind.

Dadurch werden Verknüpfungsreaktionen mit biologisch aktiven Polymeren möglich, ohne daß eine Vernetzung zwischen den Verknüpfungsreaktanten oder Mehrfachverknüpfungen erfolgen können. In der Regel steht nur eine, höchstens zwei Verknüpfungsgruppen pro Polymer zur Verfügung. Erfindungsgemäße Verbindungen ermöglichen daher einen einfachen Zugang zu solch wertvollen monofunktionellen (Reaktiv-)Polymeren.

Als Monomerbausteine für die radikalische Polymerisation kommen beispielsweise Acrylsäure-Derivate, Vinyl- oder Styryl-Verbindungen oder Mischungen davon in Frage. Dabei kann es sich beispielsweise um deren Säure-, Ester-, Amid- oder Keton-Derivate handeln. Die Monomerbausteine können reaktive oder aktivierbare Gruppen enthalten, welche die kovalente Bindung beispielsweise zu einem Chelatbildner (z.B. 1,3-Diamino-2-hydroxypropan-N,N,N',N'-tetraessigsäure) und/oder Farbstoffien (z.B. Cumarine, Fluorescine, Rhodamine) ermöglichen. Derartige Gruppen können beispielsweise Säurehalogenid-, Imidester-, Benzotriazolyl-, Isocyanato-, Isothiocyanto-, Oxiran- oder Diimid-Gruppen sein. Bevorzugte Monomerbausteine sind (Meth)Acrylsäuechlorid, (Meth)Acrylsäure-N-hydroxysuccinimidester, (Meth)Acrylsäure-N-hydroxy-phthalimidester, N-(Meth)-acryloylbenztriazol, 3- oder 4-Isothiocyanatophenyl-(meth)acrylat, 2-Isocyanatoethyl(meth)-acrylat, Isocyanatoisopropenylbenzol, Isopropenyl-α,α-dimethylbenzylisocyanat, Vinyloxiran oder eine Kombination aus (Meth)Acrylsäure mit Carbodiimiden.

Das erfindungsgemäße Verfahren wird durch die nachfolgenden Beispiele näher erläutert.

Herstellung von biologisch aktiven Initiatoren der Formel (I)

Beispiel 1 (entspricht Initiator 1)

3 mmol Biotin wird mit 1 mmol 2,2'-Azobis[2-methyl-N-(2-hydroxyethyl)propionamid] und 3 mmol Dicyclohexylcarbodiimid in 20 ml trockenem Dimethylformamid bei Raumtemperatur 18 h unter Reinstickstoff gerührt. Der entstandene Niederschlag wird abfiltriert. Dem Filtrat wird 1 ml konzentrierte wäßrige Ammoniaklösung zugesetzt. Es wird noch eine Stunde bei Raumtemperatur gerührt und nochmals filtriert. Das Filtrat wird auf zerstoßenes Eis gegossen, wobei das erfindungsgemäße Produkt (Initiator 1) ausfällt. Der getrocknete und gewaschene Niederschlag ergibt laut DC-Analyse in Methanol:Chloroform (2:1, $I_2$-Dektektion) und $^1$H-NMR das reine erfindungsgemäße Produkt.

Beispiel 2 (entspricht Initiator 6)

1 mmol p,p'-Biisocyanatobenzoylperoxid wird mit 2 mmol 1-Aminoethanol in Methylenchlorid umgesetzt. Das Methylenchlorid wird verdampft und der Rückstand wie unter Beispiel 1 beschrieben mit Biotin und Dicyclohexylcarbodiimid umgesetzt.

Beispiel 3

In 200 ml Methylenchlorid werden 10 g Azobisisobutyronitril (AIBN) sowie 100 g Polyethylenoxid (Molmasse 400), Polyol E 400 zugefügt. Die Lösung wird auf 0 °C abgekühlt und bis zur Sättigung HCl-Gas eingeleitet (ca. 46 g HCl in 3 h). Die resultierende klare Lösung wird über Nacht bei 0 °C gerührt und danach langsam unter Rühren auf 200 g Eis/100 g Wasser gegossen. Nach 2 h Rührzeit werden die beiden Phasen im Scheidetrichter getrennt, die wäßrige Phase wird 3 mal mit Methylenchlorid nachextrahiert und die vereinigten Phasen mit wäßriger $NaHCO_3$Lösung und Wasser ausgeschüttelt. Die organische Phase wird über $Na_2SO_4$ getrocknet und am Rotationsverdampfer bei Raumtemperatur im Hochvakuum eingedampft. Die Ausbeute beträgt 41,6 g, entsprechend 70,7 % der Theorie. Die $^{14}N$-Elementaranalyse ergibt 3,4 % Stickstoff (theroretisch 3,1 %).

Beispiel 4

In eine Lösung aus 5 g Produkt aus Beispiel 1 in 50 ml Tetrahydrofuran wurde bis zu Sättigung $COCl_2$ eingeleitet. Nach dem Eindampfen bis zur Trockne verbleiben 5,5 g eines viskosen Öls.

Beispiel 5 biotinylierter Starter = Initiator 2

In 15 ml Dimethylsulfoxid (DMSO) werden 1 g $Na_2CO_3$ und 1 g Biotinhydrazid zum Lösen auf 75°C erwärmt. Nach dem Abkühlen auf 0°C werden 2,02 g Substanz aus Beispiel 2 zugegeben und über Nacht bei Raumtemperatur nachgerührt. Der Niederschlag wird abfiltiert, das Filtrat am Rotationsverdampfer eingedampft, in Chloroform aufgenommen, mit $NaHCO_3$-Lösung und Wasser ausgeschüttelt und über $Na_2SO_4$ getrocknet. Nach Abdampfen des Lösungsmittels verbleiben 1,26 g eines viskosen Öls.

Beispiel 6

Herstellung von Oligonukleotid-Initiatoren

1,5 mg (0,26 µmol) des 5'-Aminolink-Oligonukleotids der Sequenz ATCCAGTTGTGTCTTCAAC in Natriumphosphatpuffer (pH = 7,5) werden mit einer Lösung von 6 mg (12,6 µmol) 4,4'-Azobis(4-cyanopentansäure N-Hydroxysuccinimidylester) in Dimethylformamid versetzt. Das Reaktionsgemisch wurde 72 h bei Raumtemperatur gerührt. Das Reaktionsprodukt wird durch präpative HPLC (Hochdruckflüssigkeitschromatographie) an einer RP 18 Säule mit einem in 30 min linar ansteigenden Gradienten von Acetonitril in 0,1 M Triethylammoniumacetat isoliert Ausbeute 35 % der Theorie.

Verwendung

Beispiel 7

Herstellung eines Markierungspolymeren aus Initiatoren mit Biotin als biologisch aktiver Endgruppe

In 10 ml trockenem Dimethylsulfoxid werden gelöst:
a) 1,5 g Natrium-p-styrylsulfonat
b) 0,5 g Cumarinfarbstoff 1 und
c) 100 mg des Initiators aus Beispiel 5
Cumarinfarbstoff der Formel:

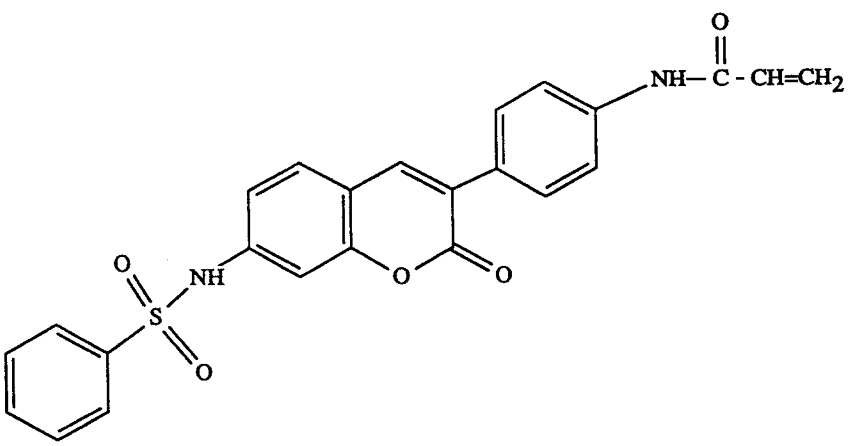

Die Lösung wird mit Reinstickstoff durchgespült, auf 70°C erhitzt und 16 h bei dieser Temperatur gehalten. Der Rohansatz wird in 200 ml Methanol gefällt, abgesaugt, getrocknet und einer Ultrafiltration (Ausschlußgrenze 10 000 Dalton) unterworfen. Das wasserlösliche, fluoreszierende Polymer hat eine mittlere Molmasse von 110.000 Dalton ($\overline{M}_n$) und kann direkt für Umsetzungen mit Avidin oder Streptavidin eingesetzt werden.

Beispiel 8

Herstellung eines Markierungspolymeren aus Initiatoren mit Oligonukleotid als biologisch aktiver Endgruppe

Es wird genauso verfahren, wie in Beispiel 7, statt des Initiators aus Beispiel 5 wird der Initiator aus Beispiel 6 und statt 10 ml Dimethylsulfoxid werden 1,2 ml verwendet:

a) 0,3 g Natrium-p-styrylsulfonat
b) 0,1 g Cumarinfarbstoff 1 (Formel vgl. Beispiel 4) und
c) 0,5 mg des Initiators aus Beispiel 6

Das Polymer hat eine mittlere Molmasse von 500 000 ($\overline{M}_n$) und kann direkt in Gensondentests zum Nachweis von DNA oder RNA der Komplimentärsequenz des Oligonukleotids eingesetzt werden

Biologisch aktive Substanzen, die mit Markierungspolymeren markiert sind.

Beispiel 9

Doppelmarkierung der Polymer-Oligonukleotidsonde

Zum Nachweis der Kopplung der Oligonukleotidsonde an das Polymer und zum Vergleich der Nachweisempfindlichkeit der im Polymer gebundenen Cumarin-Fluoreszenzfarbstoffe mit den herkömmlichen Phosphor$^{32}$ ($P^{32}$) oder Digoxigenin-Markierungen wurde eine Doppelmarkierung der Polymer-Oligonukleotidsonde durchgeführt.

Die reaktive 5' Amino-Oligonukleotidsonde mit der 19 mer Nukleotidsequenz 5'd ATCCAGTTGTGTCTTCAAC aus Beispiel 6 wurde mit alpha $P^{32}$-dCTP unter Verwendung eines Endgruppenmarkierungskits der Firma Boehringer Mannheim am 3'Ende markiert. Die Endgruppenmarkierung wurde alternativ nicht radioaktiv mit Digoxigenin-dUTP vorgenommen. In einem 50 µl Ansatz mit 10 µl Reaktionspuffer (Kaliumkakodylat 1 mol/l; Tris/HCL 125 mmol/l; Rinderserumalbumin 1,25mg/ml;-pH 6,6; 25 °C) 1-2 µg Oligonukleotid, 5 Einheiten Terminale Transferase, Kobaltchlorid ($COCl_2$) 2,5mmol/l und 25 µCi alpha$P^{32}$dCTP werden nach 60 Minuten bei 37 °C ca. 50 % Endmarkierung erreicht.

Die Kopplung an das Polymer wurde wie in Beispiel 8 beschrieben durchgeführt.

Das Polymer wurde in Ethanol gefällt und dann in 1 ml bidestilliertem Wasser gelöst. Durch Gelelektrophorese in 17 %igem Polyacrylamidgel wurde nachgewiesen, daß das Oligonukleotid an das Polymer gebunden ist.

Mit der doppelmarkierten Polymer-Oligonukleotidsonde, die einen Nachweis über die Fluoreszenz des im Polymer gebundenen Cumarin-Fluoreszenzfarbstoffes ermöglicht oder einen Nachweis über das im Oligonukleotid gebundene $P^{32}$ bzw. Digoxigenin wurde eine Slot Blot Hybridisierung wie in dem nachfolgenden Beispiel 10 beschrieben und eine Flüssighybridisierung wie im Beispiel 11 beschrieben durchgeführt.

Beispiel 10

Slot Blot Hybridisierung mit Polymer-Oligonukleotidsonde

Die Hybridisierung wurde nach üblichen Verfahren durchgeführt bei einer Inkubationstemperatur von 40 bis 68 °C. Je nach Hybridisierungstemperatur wurden unterschiedliche Substanzen zugesetzt. Dextransulfat oder andere Polymere wurden eingesetzt um die Geschwindigkeit und das Ausmaß der Hybridisierung zu erhöhen. Detergentien und Blockierungs-Reagentien wie Trockenmilch, Denhardt's Lösung, Heparin oder Natriumdodecylsulfat (im folgenden SDS genannt) wurden zugesetzt um die unspezifische Bindung der DNA an die Membran zu unterdrücken. Denaturierende Agentien wie Harnstoff oder Formamid können eingesetzt werden um die Schmelztemperatur der Hybride zu reduzieren, so daß niedrigere Hybridierungstemperaturen angewandt werden können. Drüber hinaus können durch Zugabe von heterologer DNA die nicht spezifische Bindung von Gensonden an nicht homologer DNA auf dem Blot reduziert werden.

Zur Vorbereitung der Hybridisierung wurden 50-500 ng der nicht markierten genomischen DNA von Nitrosomonas europeae zunächst 5 Minuten bei 100 °C denaturiert, auf 0 °C abgekühlt und dann auf vorbehandelte Nitrozellulose oder Nylonmembranen mit Hilfe eines Minifold II - Filtrationsgerätes der Firma Schleicher und Schüll aufgetragen und bei 80 °C 2 Stunden fixiert. Die Filter wurden in einer versiegelten

Plastik-Folientasche oder Plastikbox mit mindestens 20 ml Hybridisierungslösung pro 100 $cm^2$-Filter bei 68 °C mindestens 1 Stunde hybridisiert.

Die Lösung wurde durch 2,5 ml/100 $cm^2$-Filter Hybridisierungslösung 1 ersetzt, der 100 ng Polymer-Oligonukleotidsonde aus Beispiel zugesetzt wurden. Die Filter wurden mindestens 6 Stunden unter schwachem Schütteln bei 68 °C inkubiert.

Die Filter wurden dann 2 x 5 Minuten bei Zimmertemperatur mit mindestens 50 ml 2xSSC, 0,1%SDS pro 100 $cm^2$-Filter und 2 x 15 Minuten bei 68 °C mit 0,1xSSC, 0,1%SDS gewaschen.
Die Filter wurden dann direkt zur Detektion der hybridisierten DNA eingesetzt.

Lösungen:

| | |
|---|---|
| 20xSSC: | 3M NaCl, 0,3M Na-Citrat pH 7,0 |
| Hybridisierungslösung 1: | 5xSSC; 0,1% N-Lauroylsarcosin, Na-Salz; 0,02% SDS; 0,5% Blocking Reagenz (Boehringer Mannheim) Lösung bei 50-70 °C lösen |

Andere Hybridisierungslösungen, die ebenfalls für die Slot Blot Hybridisierung eingesetzt werden können, sind z.B.:

| | |
|---|---|
| Hybridisierungsmix 2: | 50% Formamid 7x SSC; 2x Denhardt's Lösung (100xDenhardt's: 2% Ficoll, 2% Polyvinylpyrrolidon, 2% Rinderserumalbumin) 300 µg/ml Kalbsthymus-DNA |
| Hybridisierungsmix 3: | 6xSSC; 10xDenhardt's Lösung; 50 µg Heringssperma DNA, Rinderserumalbumin 0,1% |
| Hybridisierungsmix 4: | 5xSSC; PEG; 5% Trockenmilchpulver 0,01M Natriumpyrophosphat; |

Der Nachweis erfolgte über den im Polymer der Oligonukleotidsonde gebundenen Cumarin-Fluoreszenzfarbstoff. Die fluoreszierenden Slot Blots des Filters wurden in einem Shimadzu CS930 Scanner quantitativ ausgewertet.
Durch die Doppelmarkierung des Oligonukleotids mit $P^{32}$ durch 3'Endgruppenmarkierung mit terminaler Transferase war eine Auswertung des Hybridisierungsversuches auch über Autoradiographie möglich. Das Filter wurde dazu auf einer Glasplatte fixiert und dann ein Röntgenfilm aufgelegt und 2-5 Stunden exponiert. Nach der Entwicklung des Films wurde die Schwärzung der Slot Blots quantitativ mit einem Shimadzu Scanner quantitativ ausgewertet. Alternativ wurden auch andere Nachweis-Methoden eingesetzt. Mit digoxigenierten Polymer-Oligonukleotid-Sonden wurde ein Farbstoff-Nachweis mit alkalischer Phosphatase konjugierten Antikörpern und Brom-Chlor-Indolylphosphat und Nitroblautetrazolium oder ein Chemilumineszenz-Read Out mit alkalischer Phosphatase und AMPPD 3-(2'-Spiroadamantan)-4-methoxy-4-(3''-phosphoryloxy)-phenyl-1,2-dioxetan als Substrat durchgeführt.

Durch die Doppelmarkierung war der direkte Vergleich der Sensitivität verschiedener Nachweis-Systeme möglich.

Ergebnis:

Durch die Signalamplifikation der Cumarin-Farbstoffmoleküle im Polymer (ca. 200 Farbstoffmoleküle/Polymer) der Oligonukleotidsonde konnte mit der Detektion von 1-0,1 pg DNA nahezu die gleiche Sensitivität wie mit enzymatischen Nachweismethoden erreicht werden. Durch die Verwendung eines Polymer-Trägers für die Cumarin-Farbstoffmoleküle wird eine um den Faktor 100-1 000 höhere Sensitivität erzielt als mit der herkömmlichen Markierung mit Fluoreszenzfarbstoffen über Fluoreszenz-Nukleotidbausteine.

Beispiel 11

Flüssighybridisierung mit der Polymer-Oligonukleotidsonde

Füssighybridisierungen wurden als Sandwich Hybridisierungen mit Streptavidin gecoateten magnetischen Partikeln der Firma Dynal, Hamburg zur Separation des Hybridisierungskomplexes durchgeführt.

Die Flüssighybridisierungsteste wurden als Sandwich Teste mit 100 ng 5'-biotinylierter Capture Oligonukleotidsonde mit der Nukleotidsequenz 5'dCTGCTCGTAGACAATGCGT, 100 ng Polymer-Oligonukleotidsonde wie im Beispiel 5 (Detector Gensonde) und Nitrosomonas Target-DNA in verschiedenen Konzentrationen (50 ng-1 000 ng) in einem Volumen von 50 µl durchgeführt.

Nach 10 minütigem Erhitzen auf 100 °C und anschließendem Abkühlen auf 0 °C wurden 50 µl 2x Hybridisierungsmix 1 Boehringer, Mannheim zugegeben und 1 Stunde bei 68 °C hybridisiert. Die magnetischen Beads wurden mit 1 x Hybridisierungsmix 1 vorbehandelt und nach dem Separieren über einen Magneten die Flüssigkeit abpipettiert und der Hybridisierungsansatz zugegeben und 1/2 Stunde bei Raumtemperatur unter schwacher Bewegung inkubiert. Der gekoppelte Hybridisierungskomplex wurde mit den Beads separiert, die Restflüssigkeit abpipettiert und einmal mit Puffer A (2xSSC; 0,1% SDS), dann mit Puffer B (0,1 SSC; 0,1% SDS) 2x gewaschen.

Anschließend wurden 500 µl bidest. $H_2O$ zugegeben und die Fluoreszenz der Polymer-Oligonukleotidsonde im Hybridisierungskomplex in einem Fluoreszenzphotometer bei 375 nm Anregung und 495 nm Emission gemessen.

Parallel dazu wurde die Blocking Reaktion und Antikörper-Reaktion zum Nachweis der Hybridisierung über Chemilumineszenz durchgeführt. Die mit DNA beladenen Beads wurden 1x mit 150 µl Waschpuffer (0,1M Maleinsäure, 0,1M NaCl, pH 7,5, 0,3% Tween 20) behandelt und nach dem Separieren und Abpipettieren des Waschpuffers 400 µl Puffer 2 (0,1M Maleinsäure; 0,15M NaCl, PH 7,5; 1% Blocking Reagenz (Boehringer) zugegeben. Nach 1/2 Stunde Inkubation bei Raumtemperatur wurde separiert, abpipettiert und 100 µl Antikörperkonjugat-Lösung (AK 1:10 000 in Puffer 2) zugegeben und 1/2 Stunde Raumtemperatur inkubiert, dann separiert, abpipettiert und mit 400 µl Waschpuffer behandelt 2x15 Minuten bei schwacher Bewegung. Anschließend wurde separiert und mit 150 µl Puffer 3 (0,1M Tris/HCl Puffer mit 0,1M NaCl und 50mM $MgCl_2$ pH 9,5) behandelt. Es wurde wieder separiert und mit Detektionslösung mit AMPPD 1:100 im Puffer 3 15 Minuten bei 37 °C im Wasserbad inkubiert, dann die Chemilumineszenz im Lumineszenz-Photometer bei 477 nm (Lumacounter von Lumac) gemessen.

Ergebnis

Durch die Signalamplifikation des Cumarinfarbstoffes im Polymer konnte eine deutlich höhere Sensitivität als mit direkter Fluoreszenzmarkierung der DNA erreicht werden. Die Sensitivität liegt um den Faktor 100-1000 höher. Die Detektionsgrenze erreicht mit 1 - 0,1 pg DNA nahezu die Chemilumineszenz-Sensitivität.

## Beispiel 12

### Hybridisierung der Polymer-Oligonukleotidsonde mit amplifizierter DNA

Durch diese Sensitivitätssteigerung wird der direkte und damit besonders einfache Nachweis über die Fluoreszenz der Polymer-Oligonukleotidsonde eine sehr gute Alternative im Vergleich zu den bekannten Nachweis-Techniken wie Chemilumineszenz, BromChlorindolyphosphat/Nitroblau-Tetrazolium-Farbstoffreaktion und die radioaktive Methoden.

Die Amplifikation der Target-DNA wurde nach der Polymerase Chain Reaktion (EP-A 200 362; 201 184) und alternativ nach der Hairpin Amplifikations Methode (EP-A 427 074) durchgeführt.

Zur PCR-Reaktion wurden eingesetzt 2 µg genomische DNA von Nitrosomonas europae, 2 µmol Primer 1 (5'dATCCAGTTGCTTCAAC) und Primer 2 (5'ACTGGCAGGCAGCAG), 2,5 Units Taq-Polymerase von Cetus/Perkin-Elmer sowie jeweils 200 µmol dNTPS in insgesamt 100 µl PCR-Puffer (50 mM KCl, 10 mM Tris/HCl pH 8,3, 1,5 mM $MgCl_2$, und 0,01% Gelatine. Die Amplifikation wurde in einem PCR-Prozessor der Firma Cetus/Perkin-Elmer durchgeführt.

Mit den Ansätzen wurde zunächst eine Initialschmelzung der DNA 2 Minuten, 30 Sek. bei 94 °C durchgeführt, dann pro Zyklus 1 Min. bei 94 °C die DNA denaturiert, 2 Minuten bei 40-45 °C das Primer-Annealing und 3 Minuten bei 72 °C die Primer-Extension durchgeführt. Nach 35 Zyklen wurde abschließend eine 20 Minuten-Extension bei 72 °C durchgeführt und die Ansätze bei 4 °C gekühlt.

Die amplifizierte DNA wurde 5 Minuten bei 100 °C denaturiert und dann die Ansätze sofort auf 0 °C gekühlt, 200 µl eiskaltes 20xSSC wurden zugegeben und sofort auf Nitrozellulose oder Nylonmembranen mit Hilfe eines Minifold II Filtrationsgerätes der Firma Schleicher und Schüll aufgetragen. Die DNA auf den Filtern wurde 2 Stunden bei 80 °C fixiert.

Die Slot Blot Hybridisierung mit der Polymer-Oligonukleotidsonde und der Nachweis erfolgte analog wie im Beispiel 11 beschrieben.

Ergebnis:

Durch die Kombination der Target-Nukleinsäure-Amplifikation mit der Signalamplifikation des Cumarinfarbstoffes im Polymer wurde eine Sensitivität erreicht, die die Detektion einzelner DNA-Moleküle ermöglicht. Die Auswertung über die Polymer-Fluoreszenz wird damit eine echte Alternative zu der sensitiven Chemilumineszenz-Methode. Im Gegensatz zu der enzymatischen Chemilumeszenzbildung kann die Fluoreszenz im Polymer direkt gemessen werden.

**Patentansprüche**

**1.** Biologisch aktive Initiatoren der Formel (I)

$A—L—B[L—A]_y$ (I),

worin

A ein biologisch aktiver Teil,
B ein radikalbildender Teil,
L eine Linkergruppierung und
y die Zahl 0 oder 1 bedeuten.

**2.** Biologisch aktive Initiatoren der Formel (I) gemäß Anspruch 1,
worin

A Biotin, Digitoxin, Digoxin, Digitoxigenin, Digoxigenin oder Oligonukleotide aus 1 bis 80 Nukleotidbausteinen ist,
L ausgewählt ist aus folgenden Gruppierungen:
$-SO_2-$, -COO-, $-SO_2NH-$, -CO-NH-, -NH-CO-O-, NH-CS-O-, -NH-CO-NH-, -NH-CS-NH-, -O-, -NH-, -S-.
B ausgewählt ist aus folgenden Strukturen:
1) Azostrukturen der allgemeinen Formel (V)

$R^{11} - N = N - R^{12}$ (V),

worin
$R^{11}$ und $R^{12}$ $C_1$-$C_{20}$-Alkyl, $C_3$-$C_{10}$-Cycloalkyl, $C_7$-$C_{20}$-Aralkyl oder die Gruppe

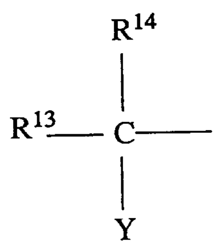

bedeuten, und
Y CN, $N_3$ oder

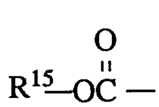 , 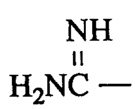 , 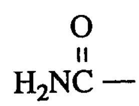 , 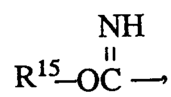

$R^{15}$-O- ,

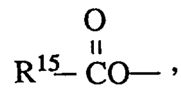

$R^{15}S$- , NCS- , SCN- ,

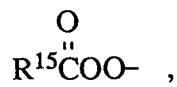

HO- ,

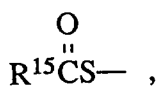

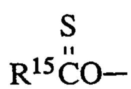 , 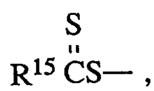 , 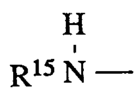 oder 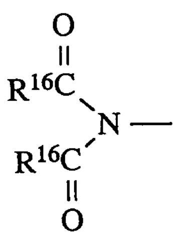

bedeutet,

$R^{13}$, $R^{14}$ und $R^{15}$ unabhängig voneinander $C_1$-$C_{20}$-Alkyl, $C_3$-$C_6$-Cycloalkyl bedeuten oder wenn $R^{13}$ und $R^{14}$ verknüpft sind $C_2$-$C_{30}$-Alkylen bedeuten oder zusätzlich einer der Reste $R^{13}$ oder $R^{14}$, jedoch nicht beide gleichzeitig, Phenyl, Tolyl, Xylyl, Benzyl oder Phenethyl bedeutet,

$R^{16}$ unabhängig $C_1$-$C_6$-Alkyl, $C_3$-$C_6$-Cycloalkyl oder $C_6$-$C_{12}$-Aryl bedeutet;

2) Tetraaryl/alkylethane der allgemeinen Formel (VI)

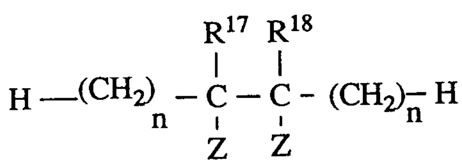 (VI),

in welcher

Z für Hydroxy, $C_1$-$C_6$-Alkyl oder $C_6$-$C_{20}$-Aryl steht,

$R^{17}$ und $R^{18}$ unabhängig voneinander für $C_1$-$C_6$-Alkyl oder $C_6$-$C_{20}$-Naphthyl, stehen und

n für die Zahlen 1 bis 6 steht;

3) Dinitrile der Formel (VII)

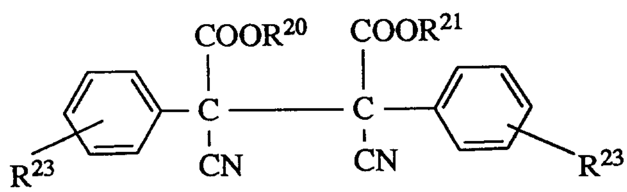 (VII),

in welcher
$R^{20}$, $R^{21}$ und $R^{23}$ unabhängig voneinander für

$$(CH_2)_m H$$

stehen, worin
m für die Zahlen 1 bis 6 steht;
4) Peroxide der allgemeinen Formel (VIII)

$$H\!-\!\left((CH_2)_l - R^{24} - \overset{O}{\overset{\|}{C}} - O\right)_2 \qquad \text{(VIII)},$$

in welcher
l die Zahlen 0 bis 6 bedeutet und
$R^{24}$ Phenylen, Naphthylen, $C_3$-$C_6$-Alkylen oder $C_3$-$C_6$-Cycloalkylen bedeutet.

**3.** Biologisch aktive Initiatoren gemäß Anspruch 1, worin
B Azostrukturen der Formel (Va) ist

$$H\!-\!\left[(CH_2)_p - \underset{Y^1}{\underset{|}{\overset{R^{25}}{\overset{|}{C}}}} - N = \right]_2 \qquad \text{(Va)},$$

in welcher
p die Zahlen 1 bis 20 bedeutet,
$Y^1$ für CN, $N_3$, $COOR^{26}$ und
$R^{25}$ und $R^{26}$ unabhängig voneinander $C_1$-$C_6$-Alkyl oder $C_3$-$C_6$-Cycloalkyl bedeuten.

**4.** Biologisch aktive Initiatoren gemäß Anspruch 1 der Formel
Initiator 1:

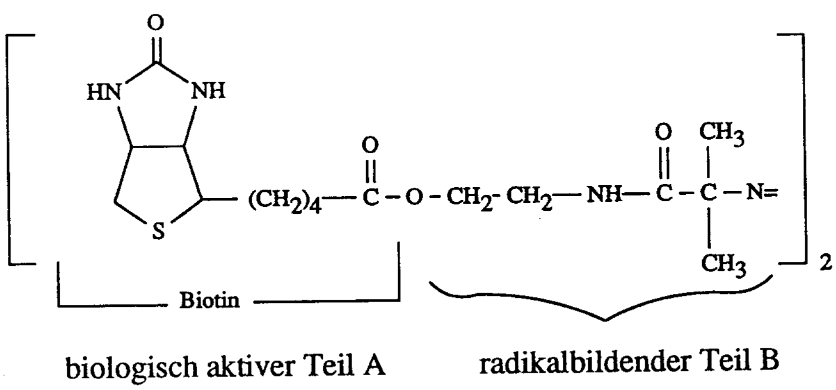


Initiator 2:

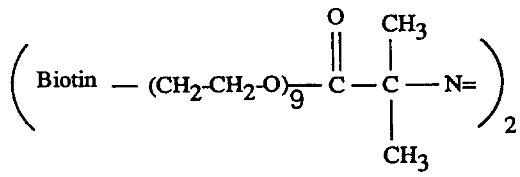

Initiator 3:

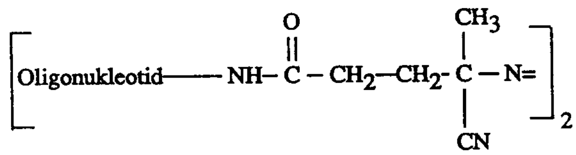

Initiator 4:

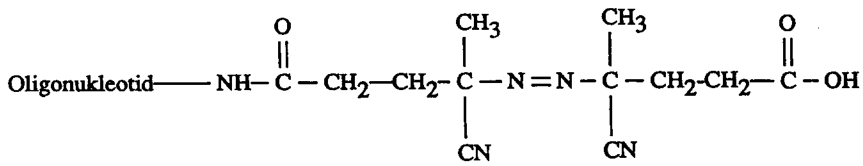

Initiator 5:

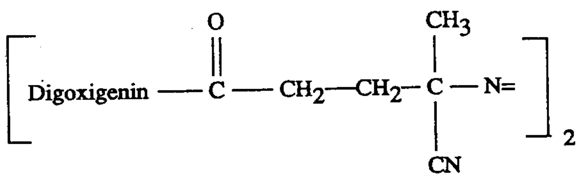

Initiator 6:

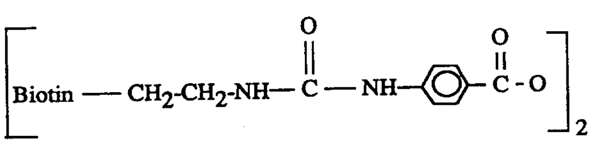

**5.** Verfahren zur Herstellung von biologisch aktiven Initiatoren der allgemeinen Formel (I)

$A—L—B[L—A]_y$ (I)

gemäß Anspruch 1, dadurch gekennzeichnet, daß man radikalbildende Verbindungen der allgemeinen Formel (IX)

$X^1—B(X^1)_y$ (IX),

wobei

B wie oben definiert ist und

$X^1$ NCO, NCS, COCl, COOH, CO-O-N-Hydroxysuccinimid, OH, $NH_2$, SH, Cl, Br oder I sein kann und

y 0 oder 1, bevorzugt 1 ist,

mit 1 oder 2 Äquivalenten biologisch aktiven Substanzen A in gegenüber den unter $X^1$ genannten Gruppierungen chemisch inerten Lösungsmitteln bei Temperaturen zwischen 0 und 40 °C umsetzt.

**6.** Verwendung von biologisch aktiven Initiatoren der Formel (I) gemäß Anspruch 1 bis 3 für die radikalische Polymerisation.